# EUROPEAN PATENT SPECIFICATION

(11) **EP 0 414 914 B1**
(45) Date of publication and mention of the grant of the patent: **11.05.1994**
(21) Application number: 90903931.5
(22) Date of filing: 28.02.1990
(51) Int. Cl.: C07H 7/06, C07H 19/02, C12P 19/12, C12P 19/28, C12N 1/20, A01N 63/02

(54) **NEW SUBSTANCE TREHALOSTATIN AND PRODUCTION THEREOF**
TREHALOSTATIN UND SEINE HERSTELLUNG
NOUVELLE SUBSTANCE, TREHALOSTATINE, ET SA PRODUCTION

(30) Priority: 28.02.1989 JP 45394/89
(43) Date of publication of application: 06.03.1991
(73) Proprietor: SUNTORY LIMITED, Kita-ku, Osaka-shi, Osaka 530 (JP)
(72) Inventor: MURAO, Sawao, Osaka 593 (JP); SHIN, Takashi, Hyogo-ken 669-13 (JP)
(74) Representative: Woods, Geoffrey Corlett
(86) International application number: PCT/JP90/00264
(87) International publication number: WO 90/10010

(56) References cited:
- GB-A- 1 544 068
- THE JOURNAL OF ANTIBIOTICS, vol. 41, no. 10, October 1988, pages 1506-1510; N. TSUJI et al.: "New glycopeptide antibiotics: II. The isolation and structures of chloroorienticins"
- THE JOURNAL OF ANTIBIOTICS, vol. 41, no. 11, November 1988, pages 1525-1532; K. DOBASHI et al.: "Novel antifungal antibiotics octacosamicins A and B. I. Taxonomy, fermentation and isolation, physico-chemical properties and biological activities"
- BIOCHEMICAL AND BIOPHYSICAL RESEARCH COMMUNICATIONS, vol. 77, no. 2, 1977, pages 449-456, Academic Press, Inc.; S.M. STRAIN et al.: "Separation of the mixture of trehalose 6,6'-dimycolates comprising the mycobacterial glycolipid fraction, "P3""
- CHEMICAL ABSTRACTS, vol. 111, no. 21, 20th November 1989, page 331, abstract no. 190010j, Columbus, Ohio, US; S. FRANZL et al.: "Purification and characterization of a beta-glucosidase (linamarase) from the hemolymph of Zygaena trifolii Esper, 1783 (Insecta, Lepidoptera)", & EXPERIENTIA 1989, 45(8), 712-18
- Agriculture and Biological Chemistry, Vol.44, No.1, (1980), Sawao Murao et al. "Isolation and Characterization of a New Trehalalase Inhibitor, S-GI"

## Description

The present invention relates to a novel substance designated Trehalostatin. The invention also relates to a process for the preparation of Trehalostatin and actinomycetes which produce Trehalostatin.

Trehalase is one of the glucohydrolases. It is an enzyme which catalyzes hydrolysis of the α-glucosidic linkage of trehalose which is widely distributed in mold, yeast and hemolymph of many species of insects.

The present invention provides a substance designated Trehalostatin, which has an inhibitory effect against trehalase in insects, which is obtainable from Amycolatopsis trehalostatica SAM 0967 (FERM BP-2784) and which:
i) is a white powder soluble in water but hardly or only slightly soluble in hexane, benzene, ethers and petroleum ether;
ii) has no absorption maxima at 220 nm or above in the ultraviolet and visible light absorption spectrum;
iii) has a positive Rydon-Smith reaction and negative ninhydrin reaction, 3,6-dinitrophthalic acid reaction and Elson-Morgan reaction;
iv) has an RF value of 0.37 in Merck Kieselgel 60 F₂₅₄ thin-layer chromatography using a 3:1:2 mixture of n-butanol, acetic acid and water as a developing solvent;
v) has an Rt of 11.0 minutes in YMC PA03 (0.7 x 27cm) high performance liquid chromatography using 65% v/v acetonitrile (in H₂O) as a solvent at a flow rate of 1.0 ml/min;
vi) has a molecular weight of 366; and
vii) has a [α]_{D} of +115°.

The present invention also provides a process for the preparation of trehalostatin which comprises culturing Amycolatopsis trehalostatica SAM 0967 (FERM BP-2784).

The present invention additionally provides Amycolatopsis trehalostatica SAM 0967 (FERM BP-2784).

The present invention further provides a culture of Amycolatopsis trehalostatica SAM 0697 (FERM BP-2784) which contains a source of assimilable carbon, a source of assimilable nitrogen and inorganic salts.

The present invention yet further provides the use of trehalostatin as an insecticide.

The present inventors found that the actinomycetes belonging to the genus Amycolatopsis, which had been separated from soil, can yield Trehalostatin. This shows an inhibitory effect against trehalase in insects, especially in Aldrichina grahami, at a very low concentration.

Amycolatopsis trehalostatica SAM 0967 has the following mycological properties.

### 1. Morphological features

SAM 0967 strain forms basal and aerial mycelium of 0.4-0.8 µm diameter. The basal mycelium branches off and is sometimes cut off. The aerial mycelium also branches off and forms around 10 or more links of spores at its end. The spores measure 0.4-0.5 µm in width and 0.9-1.3 µm in length and have a smooth surface. The structural bodies of sporangium, rhizomorph and sclerotinum were not seen even after 21 days of cultivation.

### 2. Cultural features (cultured at 28°C for 21 days)

Cultivation in a sucrose/nitrate agar medium: aerial hyphae are abundant and white in color; orangy brown in color tone of the backside; soluble pigment is grayish purple.

Cultivation in a glucose/asparagine agar medium: aerial hyphae are abundant and white in color; grayish orange in color tone of the backside; no soluble pigment.

Cultivation in a glycerin/asparagine agar medium: aerial hyphae are abundant and white in color; grayish brown in color tone of the backside; soluble pigment is orangy gray.

Cultivation in a starch/inorganic acid agar medium: aerial hyphae are abundant and white in color; whitish yellow in color tone of the backside; no soluble pigment.

Cultivation in a tyrosine agar medium: aerial hyphae are abundant; dark brown in color tone of the backside; soluble pigment is grayish brown.

Cultivation in a nutrient agar medium: aerial hyphae are abundant and white in color; whitish yellow in color tone of the backside; no soluble pigment.

Cultivation in a yeast extract/malt extract agar medium: aerial hyphae are abundant and white in color; dark brown in color tone of the backside; soluble pigment is grayish brown.

Cultivation in an oatmeal agar medium: aerial hyphae are abundant and white in color; whitish yellow in color tone of the backside; no soluble pigment.

Cultivation in an elemental agar medium: aerial hyphae are abundant and white in color; white in color tone of the backside; no soluble pigment.

Cultivation in a 1/10 potato/carrot agar medium: aerial hyphae are abundant and white; whitish yellow in color tone of the backside; no soluble pigment.

Cultivation in a 1/10 V-8 juice agar medium: aerial hyphae are abundant and white in color; whitish yellow in color tone of the backside; no soluble pigment.

### 3. Physiological features

(1) Growth temperature range
   As a result of culture tests conducted at 16°C, 19°C, 22°C, 25.5°C, 28.5°C, 31°C, 33°C, 36.5°C, 39.5°C, 42.5°C, 45.5°C and 47°C using a yeast extract/glucose liquid medium, growth of the culture was admitted to take place at temperatures above 19°C and below 39.5°C. The optimal growth temperature seemed to be around 28.5-36.5°C.
(2) Gelatin liquidization (28°C)
   Glucose/peptone/gelatin medium: positive
   Single gelatin medium: positive
   Broth gelatin medium: positive
(3) Hydrolysis of starch: negative
(4) Coagulation of skim milk (28°C): negative
(5) Peptonization of skim milk: positive
(6) Formation of melanin-like pigment
   Peptone/yeast extract/iron agar medium: negative
   Tyrosine agar medium: negative
   Tryptone/yeast extract agar medium: negative
(7) Reduction of nitrates: positive
(8) Utility of carbon sources (cultured in a test agar medium containing the following sugar at 28°C for 14 days)
   D-glucose: +
   D-xylose: +
   L-lactose: +
   L-rhamnose: +
   L-arabinose: +
   D-fructose: +
   Raffinose: ±
   D-mannitol: +
   Inositol: +
   Sucrose: +
   Lactose: +
   - (Notes) +:: utilized; ±: uncertain whether it is utilized or not; -: not utilized.
(9) Acid producing ability
   Raffinose: +
   Inositol: +
   D-mannitol: +
   Lactose: +
   Sorbitol: -
   Erythritol: -
   L-arabinose: +
   Adonitol: -
   D-galactose: +
   (Determined according to the method shown in R.E. Gordon et al: International Journal of Systematic Bacteriology, Vol. 24, P.54, 1974).

### 4. Chemical properties

### (1) 2,6-diaminopimelic acid

The presence of meso-2,6-diaminopimelic acid was recognized as a result of examination of the hydrolyzate of the whole cells and cell walls according to the method shown in J.L. Staneck and G.D. Roberts: Applied Microbiology, Vol. 28, P.226, 1974.

### (2) Saccharides

The presence of arabinose was noticed in the hydrolyzate of the whole cell. There was also admitted the presence of galactose and arabinose in the hydrolyzate of the cell wall.

### (3) Quinones

MK-9 (H4) is contained as a main constituent.

### (4) Phospholipid type

The present actinomycete strain contains phosphatidyl ethanolamine and does not contain phosphatidyl choline and an unknown glycosamine-containing phospholipid. This falls under the category of P-II type described in M.P. Lechevalier and H.A. Lechevalier: The Chemotaxonomy of Actionomycetes, PP. 227-291, in Actinomycete Taxonomy, Special Publication No. 6, Society for Industrial Microbiology (edited by A. Dietz and D.W. Thayer), U.S.A., 1980.

### (5) Mycolic acid

No mycolic acid is contained in the cell.

The cell wall of SAM 0967 strain is IV-A type containing meso-2,6-diaminopimelic acid, galactose and arbinose. SAM 0967 strain forms aerial hyphae, with links of smooth spores being formed at the end of each aerial hyphae. The quinones has MK-9 (H4) as a main component. Its phospholipid type is P-II. No mycolic acid is contained.

From the above taxonomical properties, SAM 0967 strain is identified as an actinomycete belonging to the genus Amycolatopsis. At present, 6 species and 1 subspecies are known as the species belonging to the genus Amycolatopsis. As a result of researches on the taxonomical position of SAM 0967 strain with reference to M.P. Lechevalier et al: International Journal of Systematic Bacteriology, Vol. 36, P.29, 1986, and A Henssen et al: International Journal of Systematic Bacteriology, Vol. 37, P.292, 1987, it was found that the two species Amycolatopsis orientalis and Amycolatopsis mediterranei were akin taxonomically to SAM 0967 strain. So, by using these two species as a standard strain, comparative tests were conducted with SAM 0967 strain concerning the cultural characteristics, physiological characteristics and quinones. The test results are shown in the Table below.

As seen from the Table, SAM 0967 strain is apparently distinguishable from Amycolatopsis orientalis JCM-4600 in the color tone of aerial mycelium on an yeast extract/malt extract agar medium and inorganic salt/starch agar medium, growth in the presence of 5% NaCl, utility of sucrose, acid-producing ability from raffinose, erythritol and adonitol, and menaquinone composition.

Also, SAM 0967 strain is evidently distinguishable from Amycolatopsis mediterranei JCM 4789 in aerial mycelium-forming ability and menaquinone composition.

The present inventors considered that these differences are enough to taxonomically distinguish SAM 0967 strain from said two species. Under this understanding, the present inventors concluded that SAM 0967 strain is a new species in the genus Amycolatposis and named this new species as Amycolatopsis trehalostatica.

Amycolatopsis trehalostatica SAM 0967 was deposited under FERM P-10544 at the Fermentation Research Institute of Agency of Industrial Science and Technology at the date of February 17, 1989, and later it was transferred, on February 28, 1990, to international deposition under the Budapest Treaty on the International Recognition of the Deposit of Microorganisms for the Purpose of Patent procedure and deposited under FERM BP-2784.

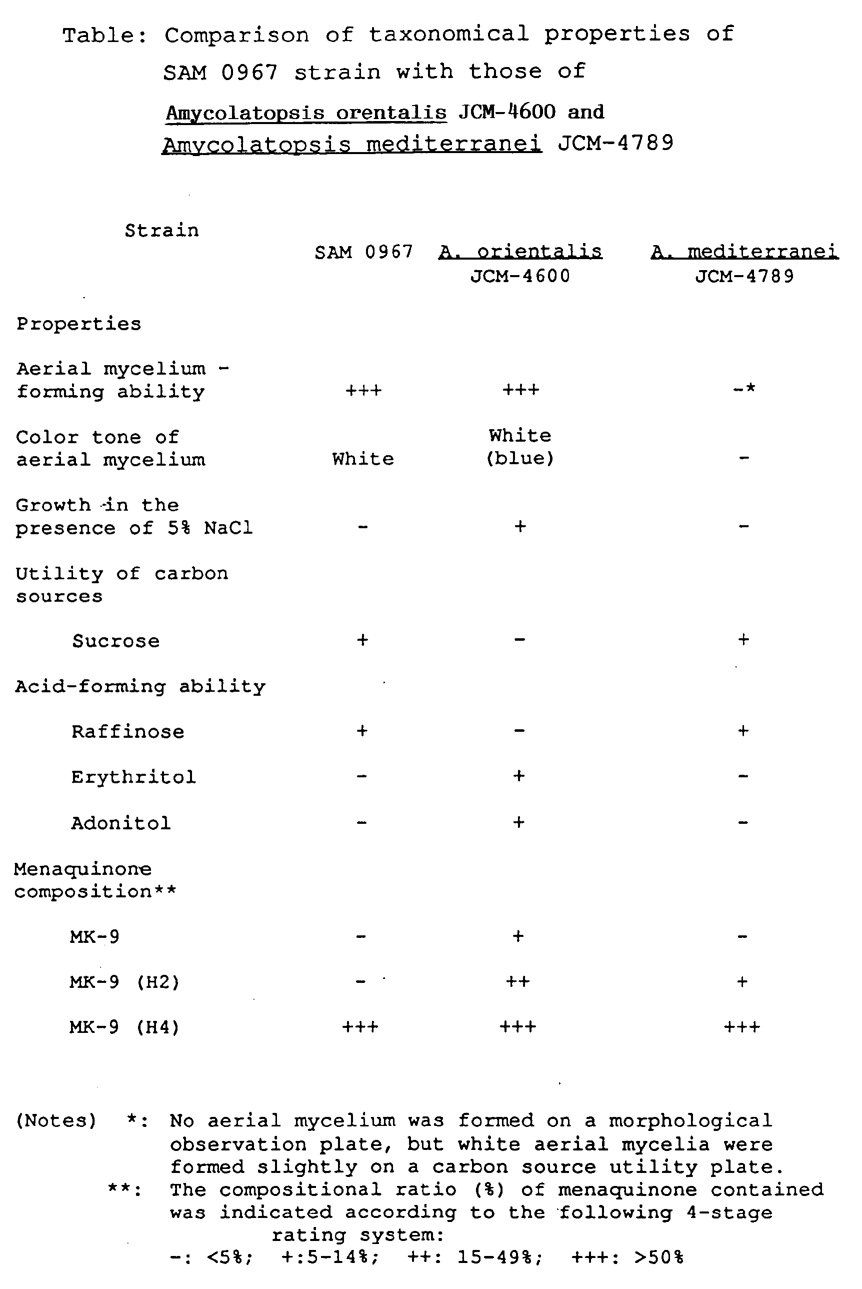

In the present invention, the actinomyces described above are cultured. The medium used for the cultivation may be either liquid or solid, but usually shaking culture or aerated spinner culture in a liquid medium is expedient.

It is possible to use any type of medium as far as it enables growth of the actinomyces according to the present invention and is capable of accumulating Trehalostatin. As carbon source, for instance, there may be used glucose, lactose, starch, sucrose, dextrin, molasses or organic acids. As nitrogen source, there may be used protein hydrolyzates such as peptone and Casamino acid, meat extract, yeast extract, soybean cake, corn steep liquor, amino acids, ammonium salts, nitrates and other organic or inorganic nitrogen compounds. Various types of phosphates, magnesium sulfate or sodium chloride, for example, may be added as an inorganic salt. Also, vitamins and nucleic acid-associated compounds may be added to promote growth of the actinomycete. In some cases, it is effective for promoting the accumulation amount of Trehalostatin to add a defoaming agent such as silicon, polypropylene glycol derivative or soybean oil to the medium.

In carrying out the cultivation in the present invention, it is desirable to initially perform a small-scale pre-cultivation and to inoculate the resulting culture into a medium to conduct the primary cultivation. In both preliminary and primary cultivation, the culturing conditions such as cultivation temperature, cultivation period and properties of the culture medium are properly selected and adjusted so as to maximize the amount of Trehalostatin accumulated in the medium. In many cases, the cultivation is preferably carried out under aerobic conditions at a temperature of 25-30°C. In case of using a liquid medium, it is recommended to maintain the pH of the medium at 5.5-8.0. In the course of such cultivation, Trehalostatin is produced and accumulated in the culture. When cultivation is carried out using the liquid medium, the objective substance is accumulated principally in a liquid phase portion of the medium, so that it is desirable to filter or centrifuge the cultures to remove the cells and then to separate the Trehalostatin from the filtrate or supernatant. In certain cases, however, the Trehalostatin may be directly separated from the liquid culture without removing the cells from them.

The detection and determination of Trehalostatin during the separation and collection can be accomplished by determining the degree to which said substance can inhibit an activity of trehalase extracted from Aldrichina grahami or porcine kidney. For the separation and purification of Trehalostatin from the cultures, there can be employed various means in accordance with the chemical characteristics of Trehalostatin. For instance, it is effective to employ treatment with an organic solvent, gel filtration by Sephadex or Biogel, ion exchange chromatography using various types of ion exchange resin, adsorption chromatography using an absorbent such as activated carbon, silica gel and Amberlite XAD-1 or XAD-2 and normal-phase chromatography using a carrier such as YMC-PA 43 or TSK-Amide 80. By using these means in a proper combination, Trehalostatin can be isolated as a white amorphous powder. Other methods than those mentioned above may also be used as far as such methods can effectively utilize the characteristics of Trehalostatin. An especially preferred combination for the absorbent is that of Dowex 50WX4 (H), YMC-PA43 and TSK-Amide 80.

Trehalostatin can be used after its isolation and purification, but in some cases, the culture of the Trehalostatin-producing actinomycete can be used in the form as it is or only after a simple purification.

The present invention is now further described in the following Examples.

### Example 1: Preparation of Trehalostatin

### (1) Activity determination method

The inhibitory activity of Trehalostatin in each stage of its purification was determined in the following way.

10 µl of an inhibitor solution and 10 µl of a trehalase solution (trehalase collected from Aldrichina grahami) were mixed. After 5-minute incubation at 37°C, 80 µl of a 5 mM trehalose and 50 mM phosphate buffer solution (pH6.5) was added, followed by an additional one-hour incubation at 37°C. Thereafter, 10 µl of a 50% trichloroacetic acid solution was added to terminate the reaction. Glucose formed in the reaction solution was determined by a new blood sugar test manufactured by Boehringer-Mannheim Ltd. The amount of the enzyme was such that absorbance of the wavelength (660 mm) used in the glucose determination when conducted according to the above method without adding the inhibitor would become 1.0. The Trehalostatin concentration which can achieve 50% inhibition of enzyme (trehalase) activity in the above determination method was expressed as 1 unit/ml.

### (2) Preparation of Trehalostatin by cultivation of

### Amycolatopsis trehalostatica

30 ml of pure culture of SAM 0967 strain was inoculated into 3.1 litres of a medium (pH 7.0) composed of hot water extract of potato, glucose, dry yeast extract, potassium monophosphate and magnesium sulfate and cultivated in a 5-liter jar fermenter at 28°C x for 120 hours (aeration, 1 vvm; agitation, 400 rpm).

To 3.07 litres of supernatant (containing 101,000 units of Trehalostatin) obtained by centrifugation of the culture, formic acid was added to a final concentration of 0.02 M. After adjusting the pH of the solution to 3.1 with hydrochloric acid, the solution was applied to a column (6 x 37cm) of Dowex 50W x 4 (H) (mfd. by Muromachi Chemical Co., Ltd.). This column was washed with 3.0 litres of 0.2 M pyridineformic acid buffer (pH 3.2) and the substance having the activity inhibiting trehalase of Aldrichina grahami was eluted in 3.5 litres of 0.4 M pyridine-acetic acid buffer (pH 4.2).

The active fraction (containing 120,000 units of Trehalostatin) was concentrated and evaporated to dryness under reduced pressure, the residue was dissolved in a small quantity of water and the pH of the solution was adjusted to 3.1 with hydrochloric acid. The insolubles in the solution were removed by a centrifuge, and the resultant supernatant was passed through a column (3 x 12cm) of Dowex 50W x 4 (H). This column was washed with 0.5 litres of deionized water and then further washed with 3 litres of 0.2 M pyridine-formic acid buffer (pH 3.2) containing 30% (v/v) of methanol to elute the objective substance.

The fraction of the objective substance (containing 120,000 units of Trehalostatin) was concentrated under reduced pressure. The residue was uniformly suspended in 50% (v/v) acetonitrile and charged into a packed column for high-performance liquid chromatography (YMC-PA 43, 2.5 x 27cm). This column was washed with a 65% (v/v) acetonitrile solution of a determined composition (flow rate: 5 ml/min), and the eluate was fractionated in portions of 5.0 ml and detected by a differential refractometer. As a result, the fraction having trehalase-inhibiting activity (containing 119,000 units of Trehalostatin) was eluted at around 200 ml after the charging.

The fraction containing the objective substance was concentrated, dissolved in 40% (v/v) acetonitrile and charged into a packed column for high-performance liquid chromatography (TSK-Amide 80, 0.7 x 27cm). This column was washed with a solvent of the same composition (flow rate: 1 ml/min) and the eluate was fractionated in portions of 0.5 ml and detected by a differential refractometer. The fraction having trehalase-inhibiting activity was eluted at around 25-30 ml after the charging. The fraction of the objective substance (containing 118,000 units of Trehalostatin) was evaporated to dryness, added with water and then freeze-dried, thereby obtaining 1 mg of a pure objective substance.

### Example 2: Physicochemical properties of Trehalostatin

The substance separated from the culture of Amycolatopsis trehalostatica (SAM 0967) and purified according to Example 1 was named Trehalostatin.

The physical and chemical properties of this Trehalostatin are shown below. 
Appearance:      white powder
Solubility:      soluble in water but hardly or only slightly
                 soluble in hexane, benzene, ethers and
                    petroleum ether
Ultraviolet and visible light absorption spectrum:
                 having no absorption maxima at 220 nm or
                 above
Color reaction: positive in Rydon-Smith reaction and
                 negative in ninhydrin reaction, 3,6-
                 dinitrophthalic acid reaction and Elson-
                 Morgan reaction
Thin-layer chromatography:
                 Rf = 0.37 (Merck Kieselgel 60 F254,
                 developing solvent: 3:1:2 mixture of n-
                 butanol, acetic acid and water)

High-performance liquid chromatography: Rt = 11.0 min.
                 (column: YMC PA03, 0.7 x 27 cm; solvent: 65%
                 v/v acetonitrile in H2O; flow rate: 1.0
                 ml/min; detection: defferential refractomer)
Molecular weight:
                 366 (m/z 367, M + H, SIMS)
[α]_{D}: +115°

### Example 3: Enzyme inhibition spectrum of Trehalostatin

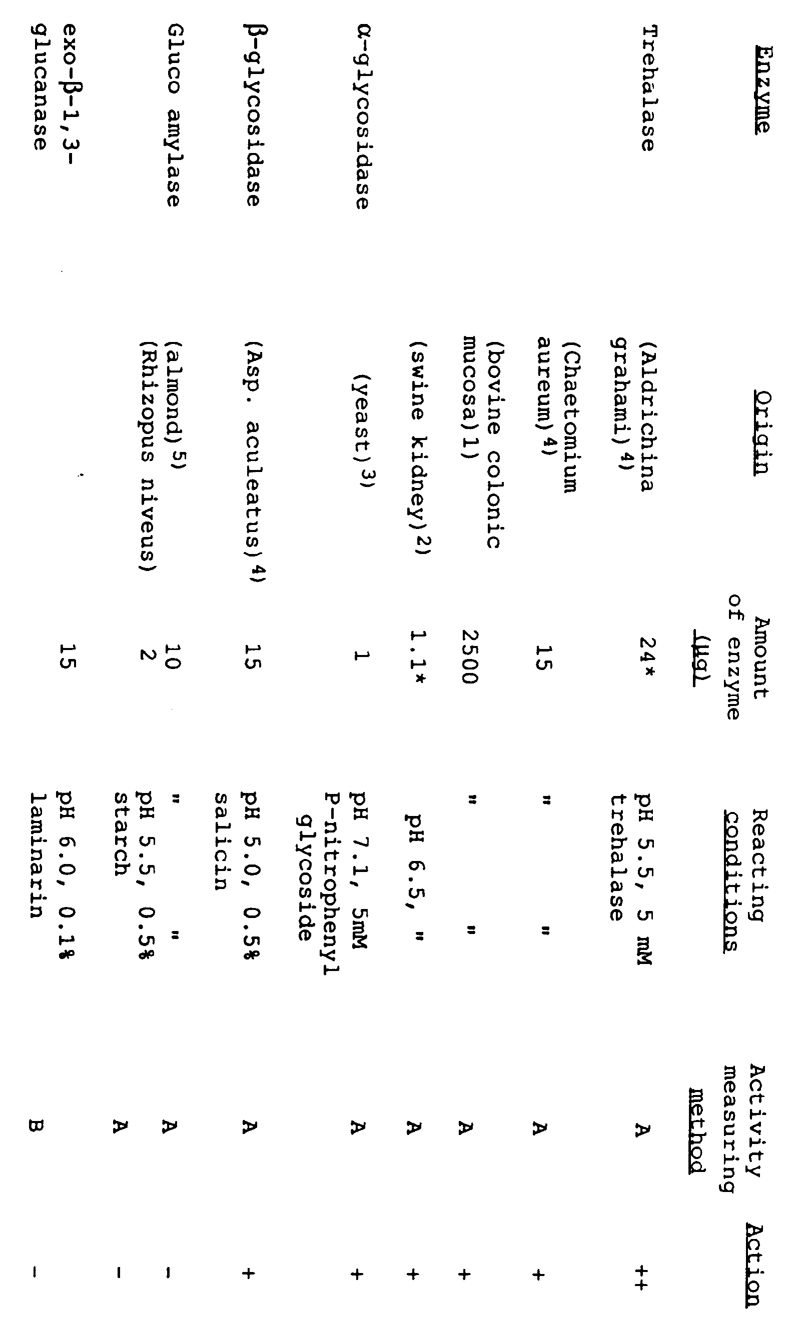

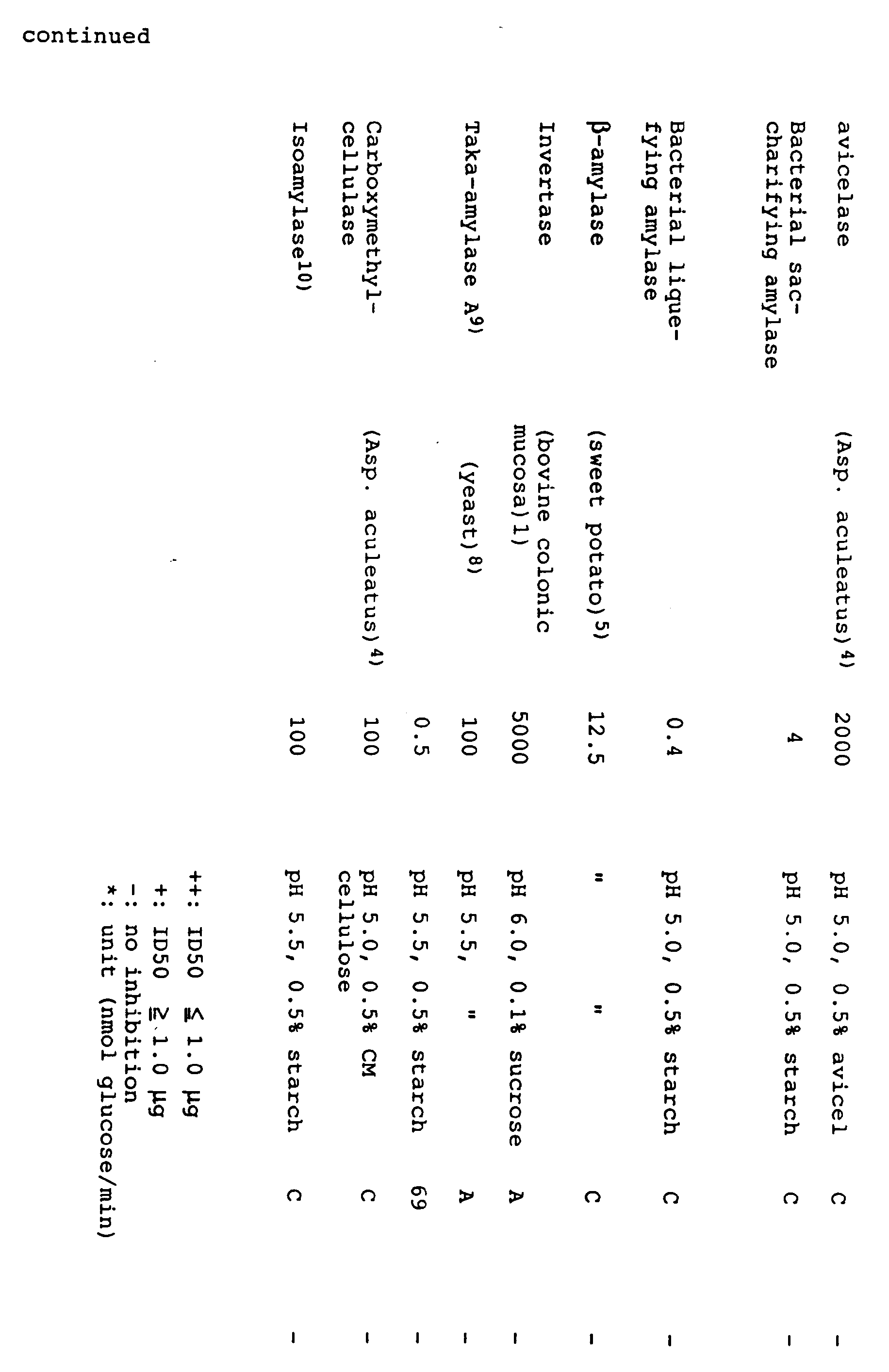

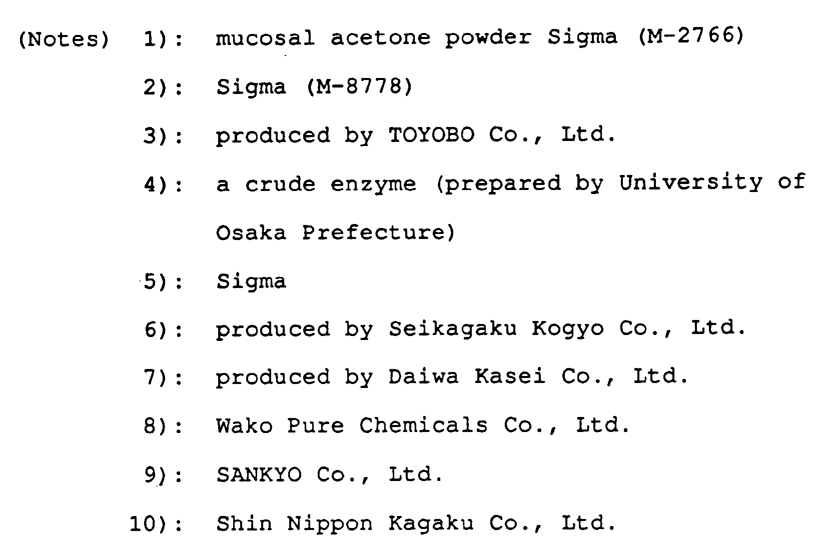

### Method:

Each enzyme (200 µl) is incubated with Trehalostatin (0.01-50 µg) in 0.05 M acetate (pH 5-6) or phosphate (pH 6-7) buffer solution at 37°C for 5 minutes. Then 20 µl of the resulting solution is collected and added with a substrate to a final concentration shown in the Table, making the total volume of the solution 100 µl. The mixture is incubated at 37°C for 10 minutes and then incubated in a boiling water bath for 3 minutes to terminate the reaction.

Determination of activity was made by measuring the increase of the product in the reaction system according to the following methods A-C.

The quantity of Trehalostatin which can effect 50% inhibition of enzyme activity under the above-described conditions was indicated as ID₅₀.
(A) The glucose produced was determined by the new blood sugar test deviced by Boehringer-Mannheim Ltd.
(B) An amount of glucose produced was determined according to the method shown in D.R. Barras and B.A. Stone: Biochim. Biophys. Acta 191, 329, 1969.
(C) The increase of an amount of reducing sugar produced was measured according to the method shown in J.A. Thoma et al: The Enzyme (3rd. ed.) 5, 115-189, 1971.

Trehalostatin exhibits an effective inhibitory effect against trehalase in insects, especially in Aldrichina grahami, even at a very low concentration and is thus useful as an insecticide for these insects.

## Claims (Claims for the following Contracting State(s): AT, BE, CH, DE, DK, FR, GB, IT, LI, LU, NL, SE)

1. A substance designated Trehalostatin, which has an inhibitory effect against trehalase in insects, which is obtainable from Amycolatopsis trehalostatica SAM 0967 (FERM BP-2784) and which:
i) is a white powder soluble in water but hardly or only slightly soluble in hexane, benzene, ethers and petroleum ether;
ii) has no absorption maxima at 220 nm or above in the ultraviolet and visible light absorption spectrum;
iii) has a positive Rydon-Smith reaction and negative nynhydrin reaction, 3,6-dinitrophthalic acid reaction and Elson-Morgan reaction;
iv) has an RF value of 0.37 in Merck Kieselgal 60 F₂₅₄ thin-layer chromatography using a 3:1:2 mixture of n-butanol, acetic acid and water as a developing solvent;
v) has an Rt of 11.0 minutes in YMC PA03 (0.7 x 27cm) high performance liquid chromatography using 65% v/v acetonitrile (in H₂O) as a solvent at a flow rate of 1.0 ml/min;
vi) has a molecular weight of 366; and
vii) has an [α]_{D} of +115°.

2. A process for the preparation of trehalostatin as defined in claim 1 which comprises culturing Amycolatopsis trehalostatica SAM 0967 (FERM BP-2784).

3. Amycolatopsis trehalostatica SAM 0967 (FERM BP-2784).

4. A culture of Amycolatopsis trehalostatica SAM 0967 (FERM BP-2784) which contains a source of assimilable carbon, a source of assimilable nitrogen and inorganic salts.

5. Use of trehalostatin as defined in claim 1 as an insecticide.

## Claims (Claims for the following Contracting State(s): ES)

1. A process for the preparation of trehalostatin, which has an inhibitory effect against trehalase in insects and which:
i) is a white powder soluble in water but hardly or only slightly soluble in hexane, benzene, ethers and petroleum ether;
ii) has no absorption maxima at 220 nm or above in the ultraviolet and visible light absorption spectrum;
iii) has a positive Rydon-Smith reaction and negative nynhydrin reaction, 3,6-dinitrophthalic acid reaction and Elson-Morgan reaction;
iv) has an RF value of 0.37 in Merck Kieselgal 60 F₂₅₄ thin-layer chromatography using a 3:1:2 mixture of n-butanol, acetic acid and water as a developing solvent;
v) has an Rt of 11.0 minutes in YMC PA03 (0.7 x 27cm) high performance liquid chromatography using 65% v/v acetonitrile (in H₂O) as a solvent at a flow rate of 1.0 ml/min;
vi) has a molecular weight of 366; and
vii) has an [α]_{D} of +115°,
which process comprises culturing Amycolatopsis trehalostatica SAM 0967 (FERM BP-2784).

2. A culture of Amycolatopsis trehalostatica SAM 0967 (FERM BP-2784) which contains a source of assimilable carbon, a source of assimilable nitrogen and inorganic salts.

## Patentansprüche (Patentansprüche für folgende(n) Vertragsstaat(en): AT, BE, CH, DE, DK, FR, GB, IT, LI, LU, NL, SE)

1. Substanz mit der Bezeichnung Trehalostatin, die eine inhibitorische Wirkung gegen Trehalase bei Insekten hat, aus Amycolatopsis trehalostatica SAM 0967 (FERM BP-2784) erhältlich ist und die:
i) ein weißes Pulver ist, das wasserlöslich, jedoch kaum oder nur gering löslich in Hexan, Benzol, Ethern und Petrolether ist;
ii) kein Absorptionsmaximum bei 220 nm oder darüber im Ultraviolett- oder sichtbaren Absorptionsspektrum hat;
iii) eine positive Rydon-Smith-Reaktion und eine negative Ninhydrin-Reaktion, 3,6-Dinitrophthalsäure-Reaktion und Elson-Morgan-Reaktion zeigt;
iv) einen RF-Wert von 0,37 bei Merck Kieselgel 60 F₂₅₄-Dünnschichtchromatographie unter Verwendung einer 3:1:2 Mischung von n-Butanol, Essigsäure und Wasser als Laufmittel hat;
v) einen Rt von 11,0 min bei YMC PA03 (0,7 x 27 cm)-Hochleistungsflüssigchromatographie unter Verwendung von 65 % V/V Acetonitril (in H₂O) als Lösungsmittel mit einer Fließgeschwindigkeit von 1,0 ml/min hat;
vi) ein Molekulargewicht von 366 hat und
vii) einen [a]_{D} von +115° hat.

2. Verfahren zur Herstellung von Trehalostatin gemäß Anspruch 1, umfassend die Kultivierung von Amycolatopsis trehalostatica SAM 0967 (FERM-BP 2784).

3. Amycolatopsis trehalostatica SAM 0967 (FERM-BP 2784).

4. Kultur von Amycolatopsis trehalostatica SAM 0967 (FERM BP-2784), die eine Quelle von assimilierbarem Kohlenstoff, eine Quelle von assimilierbarem Stickstoff und anorganische Salze enthält.

5. Verwendung von Trehalostatin gemäß Anspruch 1 als Insectizid.

## Patentansprüche (Patentansprüche für folgende(n) Vertragsstaat(en): ES)

1. Verfahren zur Herstellung von Trehalostatin, das eine inhibitorische Wirkung gegen Trehalase bei Insekten hat und das:
i) ein weißes Pulver ist, das wasserlöslich, jedoch kaum oder nur gering löslich in Hexan, Benzol, Ethern und Petrolether ist;
ii) kein Absorptionsmaximum bei 220 nm oder darüber im Ultraviolett- und sichtbaren Licht-Absorptionsspektrum hat;
iii) eine positive Rydon-Smith-Reaktion und eine negative Ninhydrin-Reaktion, 3,6-Dinitrophthalsäure-Reaktion und Elson-Morgan-Reaktion zeigt;
iv) einen RF-Wert von 0,37 bei Merck Kieselgel 60 F₂₅₄-Dünnschichtchromatographie unter Verwendung einer 3:1:2 Mischung von n-Butanol, Essigsäure und Wasser als Laufmittel hat;
v) eine Rt von 11,0 min bei YMC PA03 (0,7 x 27 cm)-Hochleistungsflüssigchromatographie unter Verwendung von 65 % V/V Acetonitril (in H₂O) als Lösungsmittel mit einer Fließgeschwindigkeit von 1,0 ml/min hat;
vi) ein Molekulargewicht von 366 hat und
vii) einen [a]_{D} von +115° hat,
umfassend die Kultivierung von Amycolatopsis trehalostatica SAM 0967 (FERM BP-2784).

2. Kultur von Amycolatopsis trehalostatica SAM 0967 (FERM BP-2784), die eine Quelle von assimilierbarem Kohlenstoff, eine Quelle von assimilierbarem Stickstoff und anorganische Salze enthält.

## Revendications (Revendications pour l'(les) Etat(s) contractant(s) suivant(s): AT, BE, CH, DE, DK, FR, GB, IT, LI, LU, NL, SE)

1. Substance désignée par tréhalostatine, qui a un effet inhibiteur sur la tréhalase des insectes, qui peut être obtenue à partir d'*Amycolatopsis* *trehalostatica* SAM 0967 (FERM BP-2784) et qui :
1) est une poudre blanche soluble dans l'eau mais très peu ou légèrement soluble dans l'hexane, le benzène, les éthers et l'éther de pétrole ;
2) n'a pas de maxima d'absorption à 220 nm ou au-dessus dans son spectre d'absorption en lumière ultraviolette et visible ;
3) a une réaction de Rydon-Smith positive et une réaction à la ninhydrine, une réaction à l'acide 3,6-dinitrophtalique et une réaction d'Elson-Morgan négatives ;
4) a un RF de 0,37 en chromatographie sur couche mince dans du gel de silice 60 F₂₅₄ de Merck en utilisant un mélange 3:1:2 de n-butanol, d'acide acétique et d'eau comme solvant de développement ;
5) a un Rt de 11,0 minutes en chromatographie liquide à hautes performances dans une colonne YMC PA03 (0,7 × 27 cm) en utilisant comme solvant de l'acétonitrile à 65 % v/v (dans H₂O) avec un débit de 1,0 ml/min ;
6) a une masse moléculaire de 366 ; et
7) a un [α]_{D} de +115 °.

2. Procédé de préparation de la tréhalostatine telle que définie dans la revendication 1, qui comprend le fait de cultiver *l'Amycolatopsis* *trehalostatica* SAM 0967 (FERM BP-2784).

3. *Amycolatopsis* *trehalostatica* SAM 0967 (FERM BP-2784).

4. Culture d'*Amycolatopsis* *trehalostatica* SAM 0967 (FERM BP-2784) qui contient une source de carbone assimilable, une source d'azote assimilable et des sels minéraux.

5. Utilisation de la tréhalostatine telle que définie dans la revendication 1 comme insecticide.

## Revendications (Revendications pour l'(les) Etat(s) contractant(s) suivant(s): ES)

1. Procédé de préparation de tréhalostatine, qui a un effet inhibiteur sur la tréhalase des insectes, et qui :
1) est une poudre blanche soluble dans l'eau mais très peu ou légèrement soluble dans l'hexane, le benzène, les éthers et l'éther de pétrole ;
2) n'a pas de maxima d'absorption à 220 nm ou au-dessus dans son spectre d'absorption en lumière ultraviolette et visible ;
3) a une réaction de Rydon-Smith positive et une réaction à la ninhidrine, une réaction à l'acide 3,6-dinitrophtalique et une réaction d'Elson-Morgan négatives ;
4) a un RF de 0,37 en chromatographie sur couche mince dans du gel de silice 60F₂₅₄ de Merck en utilisant un mélange 3:1:2 de n-butanol, d'acide acétique et d'eau comme solvant de développement ;
5) a un Rt de 11,0 minutes en chromatographie liquide à hautes performances dans une colonne YMC PA03 (0,7 X 27 cm) en utilisant comme solvant de l'acétonitrile à 65 % v/v (dans H₂O) avec un débit de 1,0 ml/min ;
6) a une masse moléculaire de 366 ; et
7) a un [α]_{D} de +115°
lequel procédé comprend la culture d'Amycolatopsis trehalostatica SAM 0967 (FERM BP-2784).

2. Culture d'Amycolatopsis trehalostatica SAM 0967 (FERM BP-2784) qui contient une source de carbone assimilable, une source d'azote assimilable et des sels minéraux.
